# EUROPEAN PATENT SPECIFICATION

(11) **EP 3 117 006 B1**
(45) Date of publication and mention of the grant of the patent: **18.07.2018**
(21) Application number: 15718247.8
(22) Date of filing: 12.03.2015
(51) Int. Cl.: C12Q 1/00, C12Q 1/32, G01N 27/327, G01N 33/98

(54) **TEST STRIP AND APPARATUS FOR MEASURING THE CONTENT OF ALCOHOL IN BLOOD, AND A METHOD FOR MEASURING THE CONTENT OF ALCOHOL IN BLOOD**
TESTSTREIFEN UND VORRICHTUNG ZUR MESSUNG DES ALKOHOLGEHALTS IM BLUT SOWIE VERFAHREN ZUR MESSUNG DES ALKOHOLGEHALTS IM BLUT
BANDELETTE D'ESSAI ET APPAREIL DE MESURE DU TAUX D'ALCOOL DANS LE SANG ET PROCÉDÉ DE MESURE DU TAUX D'ALCOOL DANS LE SANG

(30) Priority: 13.03.2014 FI 20145230
(43) Date of publication of application: 18.01.2017
(73) Proprietor: PAL Finland Oy, 70100 Kuopio (FI)
(72) Inventor: Kolehmainen, Kari, 70100 Kuopio (FI); Vartianen Ilkka, 70100 Kuopio (FI); Pyhäluoto, Rikka, 90650 Oulu (FI)
(74) Representative: LEITZINGER OY
(86) International application number: PCT/FI2015/050158
(87) International publication number: WO 2015/136154

(56) References cited:
- WO-A1-92/17778
- WO-A1-99/19507
- WO-A1-2010/077598
- US-A1- 2010 270 175
- DATABASE WPI Week 198706 Thomson Scientific, London, GB; AN 1987-039286 XP002742921, -& JP S61 294356 A (MATSUSHITA ELEC IND CO LTD) 25 December 1986 (1986-12-25)

## Description

An object of the invention is a test strip for measuring the content of alcohol in blood.
Another object of the invention is an apparatus for measuring the content of alcohol in blood, the apparatus comprising the above-mentioned test strip and a measuring device.
Yet another object of the invention is a method for measuring the content of alcohol in blood.

The apparatus according to the invention and the test strip applicable to it are any apparatus and test strip operating on the amperometric principle. Such tests are, for example, ethanol (ethyl alcohol), methanol (methyl alcohol), ethylene glycol, and their reagents suitable for measurement by the amperometric principle.

### Prior art

A widely used and known device for determining the blood alcohol content (ethanol content) of a person is an alcometer, by means of which the blood alcohol content is determined from the air exhaled by the person. Alcometers do not, therefore, directly measure the blood alcohol content, which requires a blood test.
The operation of the portable alcometers ("field alcometers") used by Finnish authorities is based on the electrochemical oxidation of the alcohol contained in the breath. In the work material cell acting as a sensor, alcohol, that is ethanol, breaks down to form acetic acid and an excess of electrons on the top surface of the cell. The electron shortage forming on the lower surface of the cell forms an electric current between the surfaces. The voltage is measured with platinum electrodes and can be read as a per mille reading indicating the ethanol content in the blood.

The currently used alcometers are in principle functional, but they and their use have some disadvantages which are discussed below.

In their field work, paramedics and the police come across people whose blood alcohol content should be measured, but who either refuse to blow into an alcometer or are unable to do so due to their condition.

When paramedics receive a call from the emergency centre, they leave for/arrive at where the patient is. If there is reason to believe that the patient is under the influence of alcohol, a breath test is taken with an alcometer, and the result obtained will determine the treatment to be started. The problem is that the breath test does not reveal the amount of alcohol in the blood. If the patient is unable (unconscious) or unwilling to blow, only primary treatment can be started. The patient has to be transported to a first-aid unit for a blood test to determine the matter.

The weaknesses of the current operations model of the paramedics are, therefore:
- a delay in starting treatment
- additional costs (extra driving, additional procedures at the first-aid unit).

The alcohol content measurement should, however, be done quickly before further treatment is initiated, because the possible alcohol content in the patient's blood is crucial information for planning further treatment. If the blood alcohol content is not immediately determined correctly, this may lead to a delay in treatment, incorrect procedures and even the loss of a patient's life. A measurement made from the breath does not always give a sufficiently reliable picture of the blood alcohol content.
The police, in turn, use alcometers as screening devices in monitoring the use of intoxicants on the road. If the screening device indicates that there is reason to believe that a driver is guilty of drunken driving, the driver is taken either to a police station for further testing with a precision alcometer or to a health centre for a blood test. If the breath test gives a zero reading although the driver can be identified as an intoxicant user, the driver is taken to a medical examination. Since the ethanol content in the blood does not always correspond to the ethanol content in the breath, the measurements made from the breath may be misleading due to the measuring arrangement. If, on the other hand, a poorly breathing person is in question, a breath test cannot be used for correct assessment of the person's condition even as a starting point.
The weaknesses of the current operations model of the police are, therefore:
- additional costs (extra driving, additional procedures at the first-aid unit)
- the confirmation done with a precision alcometer, which is expensive to use (precision alcometers are calibrated regularly and the Finnish National Institute for Health and Welfare is responsible for their quality).
Emergency vehicles are often the first to reach a patient and their staff initiates the correct treatment or refer the person into police care. In order to be able to make the correct decision, a clear picture of the ethanol content in the blood is required.
Instead of, or in addition to, an alcometer is, therefore, required a method by means of which the alcohol content of blood can be measured quickly and reliably in field conditions.

Document US 2010/077598 describes an electrochemical ethanol sensor comprising as active reagent alcohol dehydrogenase, NAD cofactor and buffer.

### Description of the invention

There is, thus, demand for an apparatus by means of which a person's/patient's blood alcohol content can be measured from the blood reliably and accurately in field conditions, regardless of whether the person/patient is conscious or unconscious.
In order to eliminate the above-mentioned weaknesses and to meet the foregoing demand, the test strip according to the invention is characterised in claim 1. In a preferred embodiment of the test strip according to the invention, the electrodes are graphite electrodes. To the reaction area of the test strip is applied saccharose and bovine serum albumin (BSA) as auxiliary substances. In the preferred embodiment, the reagents and the auxiliary substances are dried to the base, onto the electrodes. The reagents and the auxiliary substances are preferably protected with an electro-insulating protective film (e.g. polyethylene film), and that the sample opening penetrates it. The test strip according to the invention preferably includes a temperature sensor.
The apparatus according to the invention is characterised in claim 5. In a preferred embodiment of the apparatus according to the invention, the operating power of the measuring device consists of ordinary batteries, such as three AAA batteries. The measuring device according to the invention preferably has a display, for example, an LCD display, which gives the measurement result as mg/l or in per mille.

The method according to the invention is characterised in claim 8. There has long been a need in the field for the apparatus according to the invention. By means of the invention, this need can now be met. The invention is based on the unexpected observation that the alcohol oxidation reaction catalysed by the alcohol dehydrogenase enzyme (ADH) known as such, wherein ethanol reacts with nicotinamide adenine dinucleotide (NAD⁺), which acts as a coenzyme to form acetaldehyde, a reduced form of nicotinamide adenine dinucleotide (NADH) and protons (H⁺), and may be implemented in the field with a test strip. The reaction equation of this oxidation reaction is:

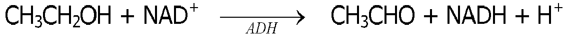

### Detailed description of the invention

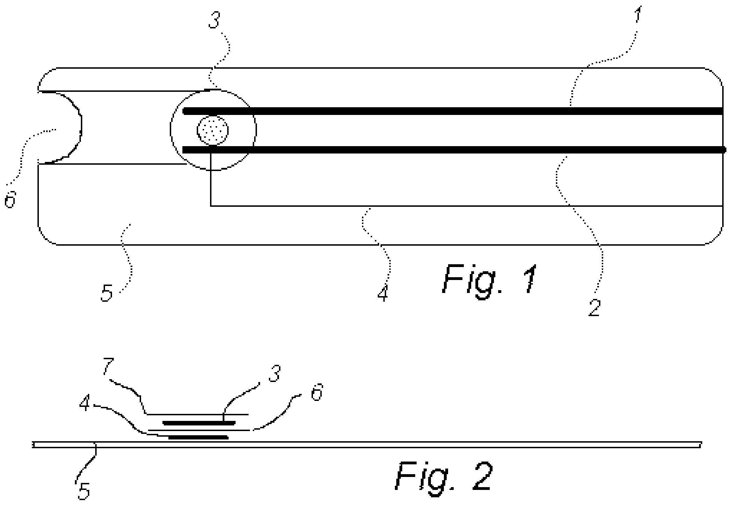

In the following is disclosed in greater detail an example of a preferred embodiment of the strip, apparatus and method according to the invention, with reference to the accompanying drawings, of which
- Figure 1: shows a top view of a preferred embodiment of the test strip according to the invention,
- Figure 2: shows a cross-sectional side view of the test strip shown in Figure 1,
- Figure 3: shows graphically the determination of the blood alcohol content from a blood sample not according to the invention.
The invention thus relates to simple and rapid blood alcohol content measurement based on a biosensor. In this preferred embodiment, the apparatus is comprised of a biosensor strip and a measuring device, the operation of which is based on measuring the difference in potential between the graphite electrodes in the biosensor, that is, the test strip, of which one is a working electrode 1 and the other a reference electrode 2. The difference in potential is formed when the ethanol contained in the sample converts to acetaldehyde and a proton (hydrogen, H⁺) in a reaction catalysed by the alcohol dehydrogenase enzyme (ADH). The apparatus contains everything needed for making the measurement. The measurement takes about 3 minutes and can be done without special training. The sample consists of blood from the fingertip, its volume being about 10 µl.
As a measuring device based on potential difference can be used a blood sugar meter applying the glucose oxidase reagent method and operating on the amperometric principle, to which meter is connected an automatic temperature sensor, from which the measuring device receives the corrective reading it uses for calculating the result. One only needs to make experimental measurements at different temperatures and include an algorithm in the program to correct the result to correspond to the room temperature. As an example of a blood sugar meter which, when modified as specified above, can be used to implement the measuring device according to the invention, can be mentioned a blood sugar meter by the name of Mendor Discreet, which is an all-in-one blood sugar meter manufactured and sold by the Finnish company Mendor Oy.
The test strip shown in Figure 1 comprises the base material 5 and two graphite electrodes 1, 2 attached to it and a reaction area 3 connected to both electrodes. To the reaction area 3 is applied nicotinamide adenine dinucleotide (NAD⁺) and alcohol dehydrogenase enzyme (ADH), and as auxiliary substances saccharose and bovine serum albumin (BSA). The reaction area 3 with its reagents and auxiliary substances is protected outwards with electro-insulating protective film 7 (e.g. polyethylene). The reaction area is connected to a sample opening 6, from which the blood sample to be measured can be transferred to the reaction area to dissolve the reagents and auxiliary substances. At the same time, the blood sample comes into contact with both electrodes 1, 2, between which can, by means of a measuring device operating on the amperometric principle, be formed a potential difference, which enables the protons (H⁺) formed in the reaction to move towards the negatively charged working electrode 1, whereby a measurable change in current is formed.

The reagents and auxiliary substances in the test strip have been added and dried onto the electrodes 1, 2. The addition of reagents and auxiliary substances to the base 5, onto the electrodes, takes place by means of industrial liquid metering, after which the reagents and auxiliary substances are dried, and finally protected with an insulating protective film 7, such as a polyethylene film.

As can be seen in Figure 2, the test strip is in general comprised of graphite electrodes 1, 2 placed on solid material, between which is formed a reaction area 3 to which the reagents and auxiliary substances have been applied in liquid form and then dried. In addition to these, the test strip also comprises a temperature sensor 4, which is positioned under the reaction area 3, separated by an insulator 6. The temperature sensor 4 is thus fixed directly to the base 5, on top of which the insulator 6 is mounted to insulate the sensor from the reaction area.

The blood alcohol content is measured as follows:
a. a blood sample is taken, for example, from a person's fingertip
b. the blood sample is brought through the sample opening of the test strip (by bringing the drop of blood into contact with the sample opening, it is absorbed in the strip) to the test strip reaction area, into contact with the reagents NADH coenzyme and alcohol dehydrogenase enzyme (ADH), whereupon the alcohol (ethanol) possibly contained in the blood sample and the NADH coenzyme react in a reaction catalysed by the alcohol dehydrogenase enzyme (NAD⁺) in such a way that the alcohol (ethanol) is converted into the corresponding aldehyde (acetaldehyde) and a proton according to the following reaction equation: 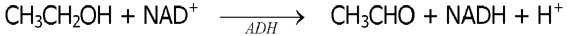
c. the test strip is connected to a device operating on the amperometric principle, which maintains the potential between the two electrodes (1, 2) constant and the instantaneous strength of the current is measured as a function of the protons produced in the reaction.
d. the magnitude of the change in the current caused by the protons produced in the reaction and obtained from the current strength measurement results is converted by means of a computation program into a value indicating alcohol content.

The measuring device operating according to the amperometric principle is a class I IVD medical device and intended for professional use. The operating power consists of three AAA batteries. The measuring device and the apparatus as a whole do not present an immediate risk to the user or patient due to the conduction of power, nor a biological risk. The apparatus includes software for controlling the use and monitoring of the apparatus. The accessories include a calibrator, control and lancets needed for taking the sample. Taking the sample requires special know-how, which must be included in the device's user requirements. The proper functioning of the device requires a temperature of 10-30°C and a relative humidity of 10-80%. For longer storage periods, the device package should be kept at room temperature (10-25°C). The shelf life of the strips is at least six months at the appropriate temperature. The measurement is done directly from the fingertip blood sample; transporting the sample elsewhere is not recommended. The measuring accuracy within the range from 0.5-6.0‰ is less than ±20 CV-%.

The properties of the measuring device
- size 100 x 50 x 30 mm
- weight 100-200 g
- product package
- device, strips, instructions for use
- implements required for sampling
- bag carried on belt
- LCD display
- result in mg/l and per mille
- date
- strip indicator
- user interface
- 2 push buttons
- menu and acknowledgement functions
- operating power 3 x AAA batteries
- sensor gate
- sensor identification
- developing the measuring device does not require special know-how
- an abundance of manufacturers available
- manufacturing costs in large production runs approximately 20-30 €
- device comprises internal calibration; external calibration is carried out with standard material
- several versions can be made of the device, e.g. equipped with data transfer
User sectors for the apparatus:
- First aid / ambulances
- Police in traffic control
- First-aid units of hospitals and health centres
- Consumer markets
- Other random users (e.g. some employers, schools, the army, etc.)

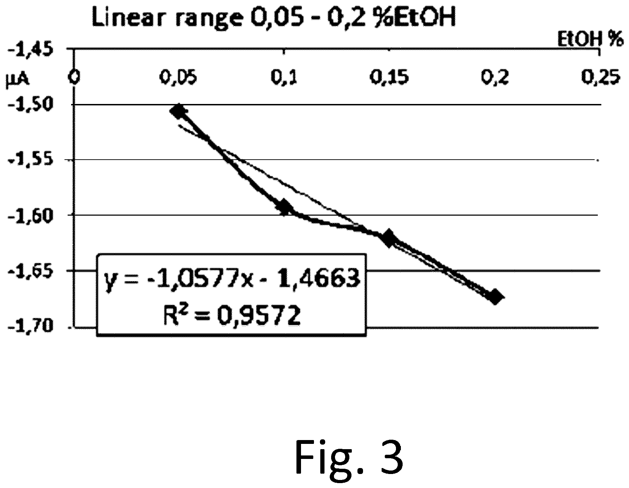

Figure 3 shows graphically the determination of the blood alcohol content from a (blood) sample not according to the invention, by using an alcohol oxidase (AOX)-Prussian blue-transmitted graphite sensor. The Figure shows the linear ratio between the alcohol (ethanol, EtOH) content and the amperometric current change by means of the so-called constant descriptor equation y = -1.0577x - 1.4663 (the square of the equation's coefficient R² = 0.9572). By means of the equation in the memory of the measuring device can reliably be calculated the ethanol content in the blood sample of the person/patient being examined.

## Claims

1. A test strip for measuring the blood alcohol content from blood, the test strip comprising a base material (5) and two electrodes (1, 2) attached to it and a reaction area (3) connected to both electrodes, to which reaction area nicotinamide adenine dinucleotide (NAD⁺) and alcohol dehydrogenase enzyme (ADH) are applied as reagents, and saccharose and bovine serum albumin (BSA) as auxiliary substances, wherein the reagents and the auxiliary substances are dried to the base (5), onto the electrodes (1, 2), a sample opening (6) in connection with the reaction area (3), from which sample opening the blood sample to be measured can be transferred to the reaction area (3) to dissolve the reagents and optional auxiliary substances, and at the same time come into contact with both electrodes (1, 2), between which can be formed a potential difference which makes possible the movement of the protons (H⁺) formed in the reaction towards a negatively charged working electrode (1), whereby a measurable change in current is formed.

2. A test strip as claimed in claim 1, wherein the electrodes (1, 2) are graphite electrodes.

3. A test strip as claimed in claim 1 or 2, wherein the reagents and auxiliary substances are protected with an electro-insulating protective film (7) (e.g. polyethylene film), and which the sample opening (6) penetrates.

4. A test strip as claimed in any of the claims 1 to 3, which comprises a temperature sensor (4).

5. An apparatus for measuring the blood alcohol content from blood, the apparatus comprising a test strip as claimed in any of the claims 1 to 4 and a measuring device with a gate for connecting the test strip to the measuring device, and configured for measuring the change in the current (potential difference) between the electrodes (1, 2) in the test strip when the test strip is connected to the gate of the measuring device.

6. An apparatus as claimed in claim 5, wherein the operating power of the measuring device consists of ordinary batteries, for example, three AAA batteries.

7. An apparatus as claimed in claim 5 or 6, wherein the measuring device has a display, for example, an LCD display, which gives the measurement result as mg/l or in per mille.

8. A method for measuring the blood alcohol content, which includes at least the following stages:
a. a blood sample is taken from a person;
b. the blood sample is brought through the sample opening of the test strip according to any of the claims 1 to 4 to the reaction area (3) thereof, into contact with the reagents NADH coenzyme and alcohol dehydrogenase enzyme (ADH), whereupon the alcohol (ethanol) possibly contained in the blood sample and the NADH coenzyme react in a reaction catalysed by the alcohol dehydrogenase enzyme (NAD⁺) in such a way that the alcohol (ethanol) is converted into the corresponding aldehyde (acetaldehyde) and a proton according to the following reaction equation: 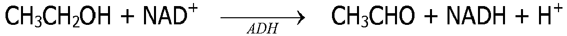
c. the test strip is connected to a device operating on the amperometric principle, which maintains the potential between two electrodes (1, 2) constant and the instantaneous strength of the current is measured as a function of the protons produced in the reaction;
d. the magnitude of the change in the current caused by the protons produced in the reaction and obtained from the current strength measurement results is converted by means of a computation program into a value indicating alcohol content.

## Patentansprüche

1. Teststreifen zum Messen des Blutalkoholgehalts aus Blut, wobei der Teststreifen ein Basismaterial (5) und zwei daran befestigte Elektroden (1, 2) und einen Reaktionsbereich (3), der mit beiden Elektroden verbunden ist, wobei zu diesem Reaktionsbereich Nicotinamidadenindinukleotid (NAD⁺) und Alkoholdehydrogenase-Enzym (ADH) als Reagenzien und Saccharose und Rinderserumalbumin (BSA) als Hilfsstoffe gegeben werden, wobei die Reagenzien und die Hilfsstoffe auf der Basis (5), auf den Elektroden (1, 2) getrocknet sind, eine Probenöffnung (6) in Verbindung mit dem Reaktionsbereich (3) umfasst, wobei die zu messende Blutprobe von der Probenöffnung zu dem Reaktionsbereich (3) überführt werden kann, um die Reagenzien und optionale Hilfsstoffe zu lösen, und gleichzeitig in Kontakt mit beiden Elektroden (1, 2) gelangen kann, zwischen denen sich eine Potentialdifferenz ausbilden kann, welche die Bewegung der bei der Reaktion gebildeten Protonen (H⁺) zu einer negativ geladenen Arbeitselektrode (1) ermöglicht, wodurch eine messbare Änderung des Stroms entsteht.

2. Teststreifen nach Anspruch 1, wobei die Elektroden (1, 2) Graphitelektroden sind.

3. Teststreifen nach Anspruch 1 oder 2, wobei die Reagenzien und Hilfsstoffe mit einem elektrisch isolierenden Schutzfilm (7) (z. B. Polyethylenfilm) geschützt sind, den die Probenöffnung (6) durchdringt.

4. Teststreifen nach einem der Ansprüche 1 bis 3, der einen Temperatursensor (4) umfasst.

5. Vorrichtung zum Messen des Blutalkoholgehalts aus Blut, wobei die Vorrichtung einen Teststreifen nach einem der Ansprüche 1 bis 4 und eine Messvorrichtung mit einem Steueranschluss zum Verbinden des Teststreifens mit der Messvorrichtung umfasst und konfiguriert ist, um die Änderung des Stroms (Potentialdifferenz) zwischen den Elektroden (1, 2) in dem Teststreifen zu messen, wenn der Teststreifen mit dem Steueranschluss der Messvorrichtung verbunden ist.

6. Vorrichtung nach Anspruch 5, wobei die Betriebsenergie der Messvorrichtung aus gewöhnlichen Batterien besteht, beispielsweise aus drei AAA-Batterien.

7. Vorrichtung nach Anspruch 5 oder 6, wobei die Messvorrichtung eine Anzeige aufweist, beispielsweise eine LCD-Anzeige, die das Messergebnis in mg/l oder in Promille angibt.

8. Verfahren zum Messen des Blutalkoholgehalts, das mindestens die folgenden Stufen umfasst:
a. einer Person wird eine Blutprobe entnommen;
b. die Blutprobe wird durch die Probenöffnung des Teststreifens nach einem der Ansprüche 1 bis 4 in dessen Reaktionsbereich (3) in Kontakt mit den Reagenzien NADH-Coenzym und Alkoholdehydrogenase-Enzym (ADH) gebracht, woraufhin der Alkohol (Ethanol), der möglicherweise in der Blutprobe enthalten ist, und das NADH-Coenzym in einer von dem Alkoholdehydrogenase-Enzym (NAD⁺) katalysierten Reaktion derart reagieren, dass der Alkohol (Ethanol) nach folgender Reaktionsgleichung in den entsprechenden Aldehyd (Acetaldehyd) und ein Proton umgewandelt wird: 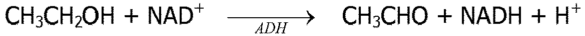
c. der Teststreifen wird mit einer nach dem amperometrischen Prinzip arbeitenden Vorrichtung verbunden, die das Potential zwischen zwei Elektroden (1, 2) konstant hält, und die momentane Stärke des Stroms wird als Funktion der bei der Reaktion erzeugten Protonen gemessen;
d. die Größenordnung der Änderung des durch die bei der Reaktion erzeugten Protonen verursachten Stroms, die aus den Ergebnissen der Stromstärkemessung erhalten wird, wird mittels eines Berechnungsprogramms in einen Wert umgewandelt, der den Alkoholgehalt angibt.

## Revendications

1. Bande d'essai permettant de mesurer la teneur en alcool sanguin à partir du sang, la bande d'essai comprenant un matériau de base (5) et deux électrodes (1, 2) attachées à celui-ci et une zone de réaction (3) reliée aux deux électrodes, auxquelles une zone de réaction de nicotinamide adénine dinucléotide (NAD⁺) et d'enzyme alcool déshydrogénase (ADH) sont appliquées comme réactifs, et du saccharose et de l'albumine de sérum bovin (BSA) comme substances auxiliaires, dans laquelle les réactifs et les substances auxiliaires sont séchés à la base (5), sur les électrodes (1, 2), une ouverture d'échantillon (6) en connexion avec la zone de réaction (3), à partir de l'ouverture d'échantillon, l'échantillon de sang peut être mesuré pour être transféré vers la zone de réaction (3) pour dissoudre les réactifs et les éventuelles substances auxiliaires, et au même moment venir en contact avec les deux électrodes (1, 2), entre lesquelles peut être formée une différence de potentiel ce qui rend possible le mouvement des protons (H⁺) formés lors de la réaction en direction d'une électrode de travail chargée négativement (1), selon laquelle un changement mesurable en terme de courant est formé.

2. Bande d'essai selon la revendication 1, dans laquelle les électrodes (1, 2) sont des électrodes en graphite.

3. Bande d'essai selon la revendication 1 ou 2, dans laquelle les réactifs et les substances auxiliaires sont protégés par un film protecteur électriquement isolant (7) (par exemple, un film de polyéthylène), et dont l'ouverture d'échantillon (6) pénètre.

4. Bande d'essai selon l'une quelconque des revendications 1 à 3, qui comprend un détecteur de température (4).

5. Appareil permettant de mesurer la teneur en alcool sanguin à partir du sang, l'appareil comprenant une bande d'essai selon l'une quelconque des revendications 1 à 4 et un dispositif de mesure muni d'une porte permettant de relier la bande d'essai au dispositif de mesure, et conçu pour mesurer le changement dans le courant (différence de potentiel) entre les électrodes (1, 2) dans la bande d'essai lorsque la bande d'essai est reliée à la porte du dispositif de mesure.

6. Appareil selon la revendication 5, dans lequel la puissance de fonctionnement du dispositif de mesure est constituée de batteries ordinaires, par exemple, de trois batteries AAA.

7. Appareil selon la revendication 5 ou 6, dans lequel l'appareil de mesure possède un affichage, par exemple, un affichage LCD, qui fournit le résultat de mesure en mg/l ou par mille.

8. Procédé permettant de mesurer la teneur en alcool sanguin, qui inclut au moins les deux étapes suivantes :
a. un échantillon sanguin est prélevé auprès d'une personne ;
b. l'échantillon sanguin est amené à travers l'ouverture d'échantillon de la bande d'essai selon l'une quelconque des revendications 1 à 4 à la zone de réaction (3) de celle-ci, en contact avec les réactifs constitués de la coenzyme NADH et de l'enzyme alcool déshydrogénase (ADH), à la suite de quoi l'alcool (éthanol) possiblement contenu dans l'échantillon sanguin et la coenzyme NADH réagissent dans une réaction catalysée par l'enzyme alcool déshydrogénase (NAD⁺) d'une manière telle que l'alcool (éthanol) est converti en l'aldéhyde correspondant (acétaldéhyde) et en un proton selon l'équation de réaction suivante : 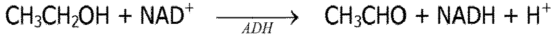
c. la bande d'essai est reliée à un dispositif fonctionnant sur un principe ampérométrique, qui maintient un potentiel constant entre deux électrodes (1, 2) et la force instantanée du courant est mesurée en tant qu'une fonction des protons produits dans la réaction ;
d. la magnitude du changement dans le courant causé par les protons produits dans la réaction est obtenue à partir des résultats de mesure de la force du courant et est convertie au moyen d'un programme de calcul en une valeur indiquant la teneur en alcool.
